# EUROPEAN PATENT APPLICATION

(11) **EP 3 597 244 A1**
(43) Date of publication of application: **22.01.2020**
(21) Application number: 18767522.8
(22) Date of filing: 15.03.2018
(51) Int. Cl.: A61M 5/32, A61M 5/28

(54) **SYRINGE ASSEMBLY**

(30) Priority: 17.03.2017 JP 2017052135
(71) Applicant: Terumo Kabushiki Kaisha, Tokyo 151-0072 (JP)
(72) Inventor: MATSUMOTO, Fumiya, Fujinomiya-shi Shizuoka 418-0004 (JP); TAKEMOTO, Masafumi, Fujinomiya-shi Shizuoka 418-0004 (JP)
(74) Representative: TBK
(86) International application number: PCT/JP2018/010263
(87) International publication number: WO 2018/169010

(57) **Abstract**

A syringe assembly (10) includes a syringe (12), a grip (16) fitted on a barrel (18) of the syringe (12), and a protective device (14) having a cover member (32) covering a needle point (27) after injection. The grip (16) has a guide tube (42) extending from an extension body (40) in a distal direction and supporting and guiding the cover member (32) from outside when the cover member (32) is axially displaced relative to the barrel (18). The guide tube (42) has an inner peripheral surface that can abut an outer peripheral surface of the cover member (32) in an initial state.

## Description

### Technical Field

The present invention relates to a syringe assembly with a protective device.

### Background Art

Syringe assemblies with a protective device for preventing inadvertent puncture due to exposure of the puncture needle before and after puncture, used for administration of liquid medicines or the like to patients are known (for example, see US 2015/0018773 A). Such a protective device has a hollow cylindrical cover member axially displaceable relative to the barrel of the syringe. Before puncture, the cover member covers the needle point of the puncture needle. The cover member is displaced in the proximal direction relative to the barrel by being pressed against the skin as the puncture needle is inserted into the skin.

### Summary of Invention

However, in conventional syringe assemblies with the protective device, clearance between components causes the cover member to shake. At the time of use, this can give a sense of unease to the user, and can cause the puncture needle to come into contact with the inner surface of the cover member. On the other hand, if the cover member is extended in the proximal direction to prevent the shaking, ease of use is deteriorated because the area held by the user at the time of use is limited. Further, the cover member extended in the proximal direction covers the barrel body, thus degrading the visibility of a liquid medicine.

The present invention has been made in view of these problems. It is an object of the present invention to provide a syringe assembly capable of preventing a protective device from shaking at least without degrading ease of use.

In order to achieve the above object, the present invention includes a syringe having a puncture needle with a needle point and a barrel holding the puncture needle, a hollow cylindrical grip fitted on an outer peripheral portion of the barrel, and a protective device having a hollow cylindrical cover member axially displaceable relative to the barrel, the protective device being configured such that the cover member covers at least part of the puncture needle in an initial state, the cover member is displaced in a proximal direction relative to the barrel by being pressed against an object to be punctured when the puncture needle is inserted into the object to be punctured, and the cover member covers the needle point after insertion of the puncture needle into the object to be punctured, in which the grip has a base provided with outwardly protruding finger-rests, an extension body protruding from the base in a distal direction, and a guide tube extending from the extension body in the distal direction and supporting and guiding the cover member from outside when the cover member is axially displaced relative to the barrel, and the guide tube has an inner peripheral surface that can abut an outer peripheral surface of the cover member in the initial state.

According to the syringe assembly of the present invention having the above configuration, the cover member of the protective device is supported from outside, so that the cover member can be prevented from shaking. Further, an area that can be held by the user at the time of use can be provided, thus allowing for various ways of holding, and does not deteriorate ease of use. Therefore, according to the syringe assembly of the present invention, the cover member can be prevented from shaking without degrading ease of use.

The guide tube may cover the cover member around an entire circumference thereof at least when supporting and guiding the cover member from outside.

This configuration can prevent shaking of the cover member from all directions around it.

The guide tube may be provided with at least one side hole through which to expose the barrel to make an inside of the barrel visible.

In the syringe assembly of the present invention, it is not necessary to lengthen the cover member in the proximal direction to prevent its shaking, so that degradation in the visibility of a liquid medicine caused by the cover member does not occur. Further, the provision of the at least one side hole in the guide tube can facilitate the visual recognition of the liquid medicine in the barrel. Thus, the cover member can be prevented from shaking without degrading the visibility of the liquid medicine.

The at least one side hole may include a plurality of side holes spaced in a circumferential direction.

This configuration can improve the visibility of the liquid medicine.

A most distal end of the guide tube may be located distally relative to a most proximal end of the cover member in the initial state of the protective device.

This configuration can favorably prevent the cover member from shaking from the beginning of pressing the cover member against an object to be punctured since the guide tube covers at least part of the cover member in the initial state of the protective device.

A most distal end of the guide tube may be located in an axial position substantially the same as a position of a distal end face of the cover member when the cover member is displaced most in the proximal direction relative to the barrel by being pressed against the object to be punctured.

This configuration allows the length of the guide tube to be increased to the maximum extent possible without impairing the function of the protective device. Thus, the cover member can be more effectively prevented from shaking.

A proximal end of the guide tube may be located proximally relative to a proximal end position of the cover member when the cover member is displaced most in the proximal direction relative to the barrel by being pressed against the object to be punctured.

This configuration can reliably prevent the shaking of the cover member until the cover member is displaced most in the proximal direction, and can reliably provide an area that can be held by the user at the time of use.

An outer diameter of a proximal end of the guide tube may be larger than an outer diameter of a distal end of the extension body.

This configuration forms a step due to the difference in outer diameter between the proximal end of the guide tube and the distal end of the extension body. This can effectively prevent the user's fingers holding the extension body from sliding in the distal direction when the user presses the syringe assembly in the distal direction to insert the puncture needle into an object to be punctured.

The grip may have engaging projections that engage a proximal end face of a flange provided at a proximal end of the barrel, thereby restricting displacement of the grip in the distal direction relative to the barrel.

This configuration can effectively prevent the grip from being displaced in the distal direction relative to the barrel.

The engaging projections may be provided at protruding pieces protruding from a proximal end face of the base in the proximal direction.

This configuration allows the engaging projections to easily elastically deform outward when the grip is fitted onto the barrel. Thus, the grip can be easily fitted onto the barrel.

The syringe assembly may further include a gasket slidably inserted in the barrel, a plunger connected to the gasket or connectable to the gasket, and a liquid medicine with which a liquid chamber formed by the barrel and the gasket is filled.

According to the syringe assembly of the present invention, the protective device can be prevented from shaking without degrading ease of use.

### Brief Description of Drawings

Fig. 1 is a perspective view of a syringe assembly (before puncture) according to an embodiment of the present invention.
Fig. 2 is a cross-sectional view taken along line II-II in Fig. 1.
Fig. 3 is a cross-sectional view taken along line III-III in Fig. 2.
Fig. 4 is a perspective view of a proximal end portion of a grip as viewed from the proximal side.
Fig. 5 is a perspective view of the syringe assembly at the time of puncture.
Fig. 6 is a perspective view of the syringe assembly after puncture.

### Description of Embodiments

Hereinafter, a preferred embodiment of a syringe assembly according to the present invention will be described with reference to the accompanying drawings. Note that the term "user" in the following description refers to a person who administers a liquid medicine (injection solution) to a patient, but the user is not limited to a doctor, a nurse, or the like, and includes the patient himself. The term "proximal end" in the following description refers to an end in a position close to the user operating the syringe, and the term "distal end" refers to an end in a position apart from the user.

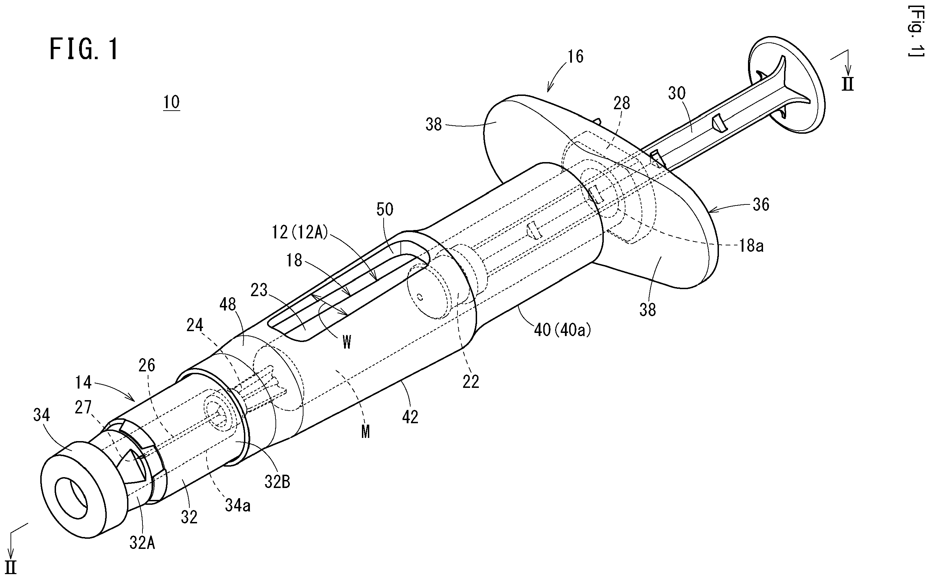

In Fig. 1, a syringe assembly 10 according to the present embodiment includes a prefilled syringe 12A as a syringe 12, a protective device 14 attached to the prefilled syringe 12A, and a grip 16 fitted on the prefilled syringe 12A.

The prefilled syringe 12A includes a hollow barrel 18 constituting the body of the syringe 12, a liquid medicine M with which the barrel 18 is filled, a gasket 22 inserted in the barrel 18, a needle holder 24 provided at the distal end of the barrel 18, and a puncture needle 26 held by the needle holder 24.

The barrel 18 is a hollow body having a substantially cylindrical barrel body 23 and having a proximal opening 18a at its proximal end. The barrel 18 is made of a material having transparency so that the inside (the liquid medicine M with which it is filled) can be visually recognized from outside. On the proximal outer periphery of the barrel 18, a flange 28 is formed, protruding radially outward. The needle holder 24 is provided at the distal end of the barrel body 23. The needle holder 24 is reduced in diameter relative to the barrel body 23, and extends in the distal direction from a distal-end central portion of the barrel body 23, holding a proximal end portion of the puncture needle 26. The flange 28 and the needle holder 24 are integrally formed with the barrel body 23.

The gasket 22 is inserted into the barrel 18 through the proximal opening 18a. The gasket 22 liquid-tightly seals the proximal side of the barrel 18, sealing the liquid medicine M in the barrel 18.

The gasket 22 is in liquid-tight contact with an inner peripheral surface of the barrel 18 at its outer periphery, and is disposed slidably in the barrel 18. A distal end portion of a plunger 30 is connected to the gasket 22. The user presses the plunger 30 so that the gasket 22 slides in the barrel 18. The plunger 30 may be connected to the gasket 22 when the puncture needle 26 is inserted into the patient to administer the liquid medicine M to the patient.

The puncture needle 26 is a hollow body in which a lumen as a fluid passage is formed, and has a sharp needle point 27 formed at its distal end. The puncture needle 26 protrudes from the needle holder 24 in the distal direction. The lumen of the puncture needle 26 communicates with a hollow portion (liquid chamber) of the barrel 18 through a proximal opening of the puncture needle 26. The liquid medicine M contained in the hollow interior of the barrel 18 is discharged from the distal end of the lumen of the puncture needle 26 and administered to the patient.

The protective device 14 covers the puncture needle 26 before and after insertion of the puncture needle 26 into the patient in order to prevent inadvertent puncture other than when the liquid medicine M is administered to the patient. The protective device 14 includes at least a hollow cylindrical cover member 32 axially displaceable relative to the barrel 18.

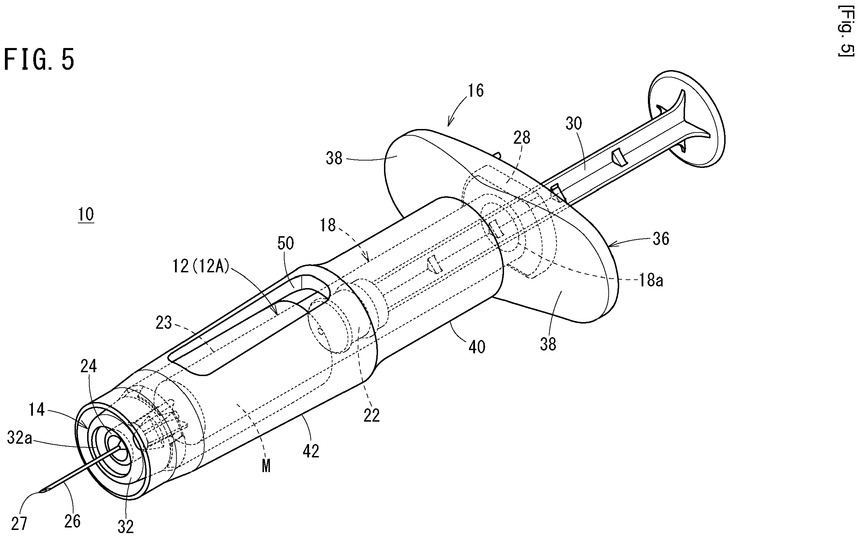

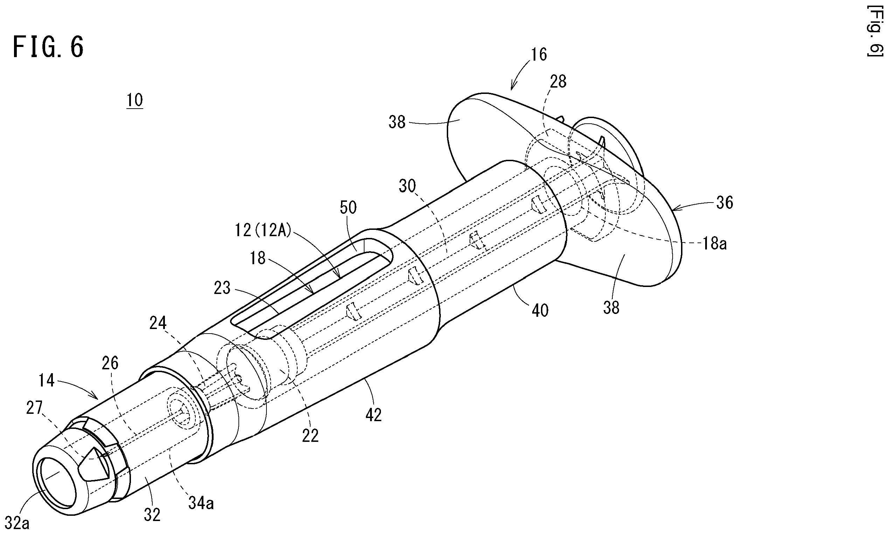

In an initial state of the cover member 32 before use shown in Fig. 1, its distal end is located distally relative to the needle point 27 of the puncture needle 26, covering the needle point 27. When the puncture needle 26 is inserted into a patient to be injected, the cover member 32 is displaced in the proximal direction relative to the barrel 18 to allow the puncture needle 26 to protrude in the distal direction from a distal opening 32a of the cover member 32 (see Fig. 5). After injection, the cover member 32 is displaced in the distal direction relative to the barrel 18 to cover the needle point 27 of the puncture needle 26 again (see Fig. 6). Note that in the initial state, the needle point 27 of the puncture needle 26 may protrude in the distal direction from the distal opening 32a of the cover member 32.

The configuration of the protective device 14 with the cover member 32 acting like this is not limited to a particular configuration, and may take various forms. Therefore, the protective device 14 may be configured as described below, for example, or may be configured in other forms.

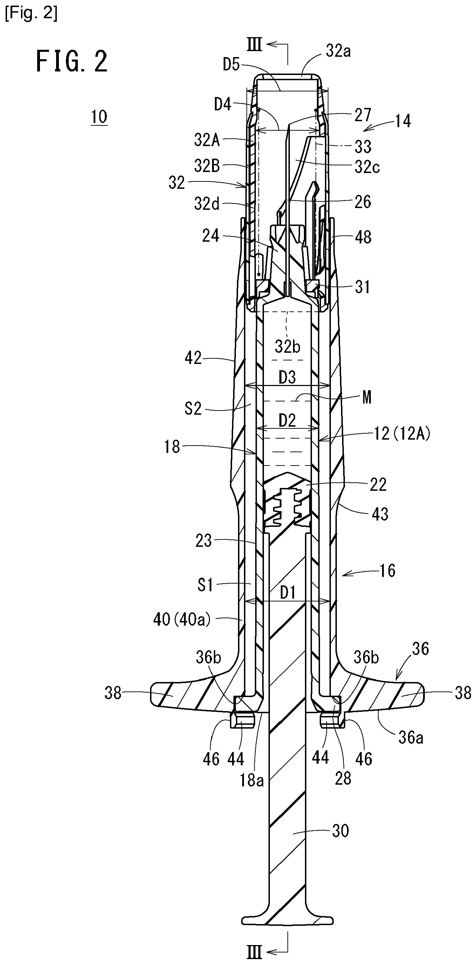

As shown in Fig. 2, the protective device 14 may include an inner member 31 rotatably fitted on the outer periphery of the needle holder 24, the cover member 32 axially movable relative to the inner member 31, and a spring member 33 (biasing member) biasing the cover member 32 in the distal direction. The cover member 32 is made of a hollow body having a side wall 32d forming a through hole 32c extending from the distal end to the proximal end. The inner member 31 is accommodated in the through hole 32c. The cover member 32 has an inner cover 32A having the through hole 32c, and an outer cover 32B fixed to an outer peripheral surface of the inner cover 32A. The inner diameter D4 of the cover member 32 is slightly larger than the outer diameter D2 of the barrel body 23.

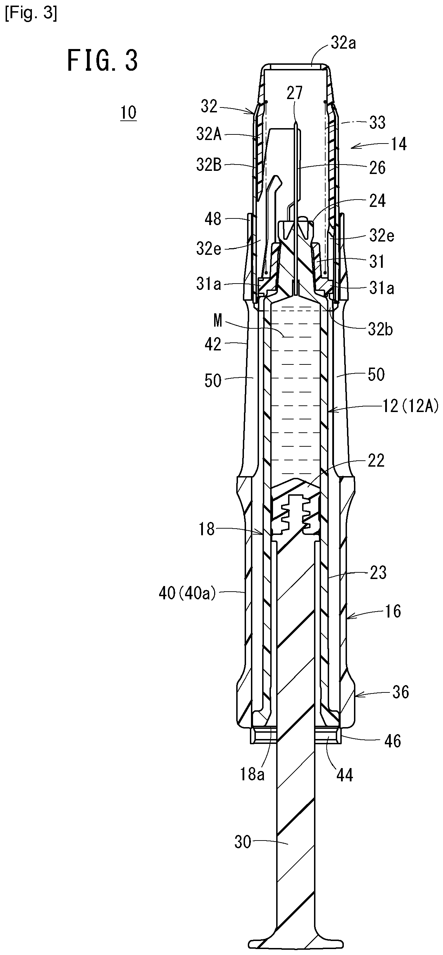

In the initial state of the protective device 14, the most proximal end of the cover member 32 is located in substantially the same axial position as the distal end of the barrel body 23. As shown in Fig. 3, a cam projection 31a protruding radially outward is formed on an outer peripheral portion of the inner member 31. A guide path 32e is formed on the side wall 32d of the cover member 32. The cam projection 31a of the inner member 31 is movably disposed in the guide path 32e. The guide path 32e is provided on the inner cover 32A. The spring member 33 is accommodated in the through hole 32c of the cover member 32, biasing the cover member 32 in the distal direction relative to the inner member 31. Note that in Figs. 2 and 3, a cap 34 (Fig. 1) is not illustrated.

The guide path 32e may be configured such that with an insertion operation of the puncture needle 26 into the patient, the cover member 32 is displaced from the initial position in the proximal direction relative to the barrel 18, guided to a retracted position, and with a removal operation of the puncture needle 26, the cover member 32 is displaced from the retracted position in the distal direction relative to the barrel 18, guided to a locked position. In this case, the cover member 32 covers the needle point 27 of the puncture needle 26 in the initial position. In the retracted position, the cover member 32 allows the puncture needle 26 to protrude from the distal opening 32a. In the locked position, the cover member 32 covers the needle point 27 of the puncture needle 26. When the cover member 32 moves from the initial position to the retracted position, the spring member 33 is axially compressed. When the puncture needle 26 is removed, the cover member 32 is moved from the retracted position to the locked position by the elastic biasing force of the spring member 33. Note that when the cover member 32 is located in the initial position, the needle point 27 may be exposed (protruded) from the distal opening 32a of the cover member 32.

Also, in this case, while the cover member 32 moves from the initial position through the retracted position to the locked position, the inner member 31 is rotated relative to the cover member 32 by the cam projection 31a moving in the guide path 32e. The inner member 31 restricts the displacement of the cover member 32 in the proximal direction relative to the inner member 31 when the cover member 32 has moved to the locked position.

In Fig. 1, the cap 34 covers the puncture needle 26. The cap 34 is made of a flexible resin material such as a rubber material, and has a sealing portion 34a into which the puncture needle 26 is inserted. Before use of the syringe assembly 10, the cap 34 is inserted in the cover member 32, covering the puncture needle 26 to seal the needle point 27 (the distal opening of the puncture needle 26). At the time of use of the syringe assembly 10, the cap 34 is removed by the user pinching its distal end portion and pulling it out in the distal direction.

Next, the configuration of the grip 16 will be described. In the present embodiment, in the product offering state of the syringe assembly 10, as shown in Fig. 1, the grip 16 is fitted on a proximal outer peripheral portion of the prefilled syringe 12A. Note that in the product offering state, the grip 16 may be separated from the prefilled syringe 12A, and fitted on the proximal outer peripheral portion of the prefilled syringe 12A when the user uses it.

The grip 16 includes a base 36 having a pair of finger-rests 38, an extension body 40 protruding in the distal direction from the base 36, and a guide tube 42 extending in the distal direction from the extension body 40.

As shown in Fig. 2, the base 36 is a portion constituting the proximal end of the grip 16, and is formed in a plate shape orthogonal to the axis of the grip 16. The pair of finger-rests 38 at the base 36 protrude in a flange shape in opposite directions perpendicular to the axis of the grip 16. Thus, the shape of the base 36 as viewed from the axial direction is relatively long in the protruding direction of the finger-rests 38, and is relatively short in the direction perpendicular to the protruding direction. With the grip 16 fitted on the barrel 18, the pair of finger-rests 38 protrude outward beyond the flange 28.

The base 36 has a proximal end face 36a provided with two flange housings 36b in a stepped shape (recessed shape) having a depth in the axial direction. The two flange housings 36b are provided in positions opposite to each other with respect to the axis of the grip 16. Two end portions of the flange 28 in the long axis direction are housed in the two flange housings 36b, individually. The depth of the flange housings 36b is set substantially the same as the thickness of the flange 28.

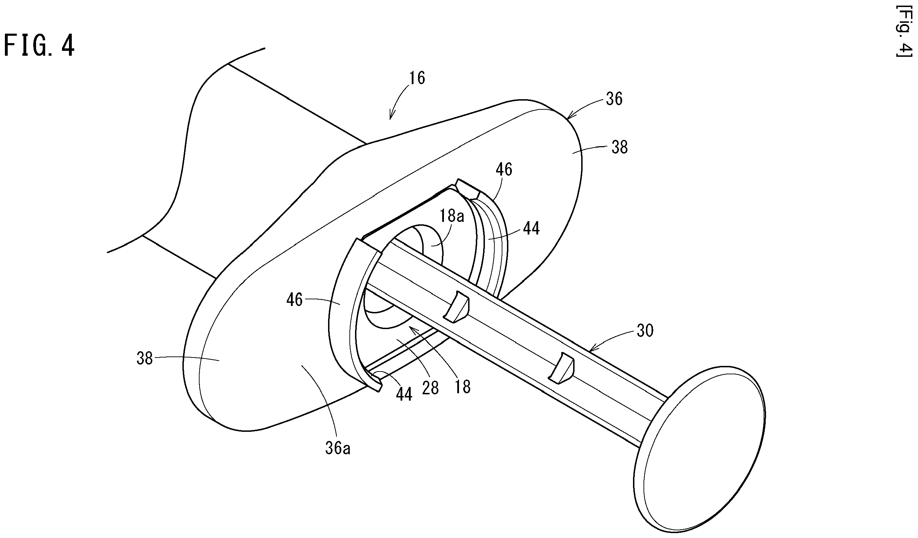

The grip 16 has engaging projections 44 that engage a proximal end face of the flange 28 provided at the proximal end of the barrel 18, thereby restricting the displacement of the grip 16 in the distal direction relative to the barrel 18. The engaging projections 44 bulge inward (toward the axis of the grip 16). As shown in Figs. 2 and 4, in the present embodiment, the engaging projections 44 are provided on the inner surfaces of two protruding pieces 46 protruding in the proximal direction from the proximal end face 36a of the base 36. The protruding pieces 46 are provided in an arc shape along the flange housings 36b, and are provided opposite to each other with respect to the axis of the grip 16. The engaging projections 44 are configured to engage the two end portions of the flange 28 in the long axis direction.

As shown in Fig. 2, with the grip 16 fitted on the barrel 18, the flange 28 of the barrel 18 is disposed in the flange housings 36b, and is locked by the engaging projections 44. Thus, the axial movement of the grip 16 relative to the barrel 18 is restricted.

As shown in Figs. 1 and 2, the extension body 40 is formed in a hollow cylindrical shape protruding in the distal direction from the base 36. As shown in Fig. 2, the inner diameter D1 of the extension body 40 is larger than the outer diameter D2 of the barrel body 23. Thus, a space S1 in an annular shape extending in the axial direction is formed between the inner peripheral surface of the extension body 40 and the outer peripheral surface of the barrel body 23. Note that the inner diameter D1 of the extension body 40 may be set substantially the same as the outer diameter D2 of the barrel body 23 so that the barrel body 23 is supported by the inner peripheral surface of the extension body 40.

The guide tube 42 supports and guides the cover member 32 from outside when the cover member 32 is axially displaced relative to the barrel 18. An inner peripheral surface of the guide tube 42 can abut an outer peripheral surface of the cover member 32 in the initial state. The guide tube 42 is formed in a hollow cylindrical shape. The most distal end of the guide tube 42 (distal opening 32a) is located distally relative to the most proximal end of the cover member 32 (a proximal opening 32b) in the initial state of the protective device 14 shown in Figs. 1 and 2. More specifically, the most distal end of the guide tube 42 is located in substantially the same axial position as the position of the distal end face of the cover member 32 when the cover member 32 is displaced in the proximal direction relative to the barrel 18 by being pressed against an object to be punctured (see Fig. 5).

In Fig. 2, the most distal end (distal opening 32a) of the guide tube 42 is located distally relative to the distal end face of the needle holder 24.

The guide tube 42 is configured to cover the cover member 32 around its entire circumference at least when supporting and guiding the cover member 32 from outside. Specifically, the guide tube 42 has an annular guide 48 surrounding the entire circumference of the cover member 32. The annular guide 48 constitutes a distal end portion of the guide tube 42.

In the initial state of the protective device 14, the annular guide 48 covers a proximal-side area of the cover member 32. The area (axial length) over which the annular guide 48 covers the cover member 32 in the initial state of the protective device 14 is, for example, one fourth or more of the total length of the cover member 32 (the length from the distal opening 32a to the proximal opening 32b), and preferably, one third or more of the total length of the cover member 32.

The inner diameter D3 of the guide tube 42 is larger than the outer diameter D2 of the barrel body 23. Thus, a space S2 in an annular shape extending in the axial direction is formed between the inner peripheral surface of the guide tube 42 and the outer peripheral surface of the barrel body 23. The space S1 in the extension body 40 communicates with the space S2 in the guide tube 42. In Fig. 2, the inner diameter D3 of the guide tube 42 is constant along the axial direction, and is set equal to the inner diameter D1 of the extension body 40. Note that the inner diameter D3 of the guide tube 42 may be different from the inner diameter D1 of the extension body 40.

The inner diameter D3 of the guide tube 42 is set slightly larger than the outer diameter D5 of the cover member 32 so that the guide tube 42 does not hinder the axial movement of the cover member 32 while stably guiding the axial movement of the cover member 32. The ratio of the inner diameter D3 of the guide tube 42 to the outer diameter D5 of the cover member 32 is set, for example, to 100 to 115%, and is preferably set to 100 to 103%.

In Fig. 2, the outer diameter of the guide tube 42 decreases in the distal direction. Accordingly, the thickness of the guide tube 42 decreases in the distal direction. Note that the outer diameter of the guide tube 42 may be constant along the axial direction. In Fig. 2, the outer diameter of the proximal end of the guide tube 42 is larger than the outer diameter of the distal end of the extension body 40. A portion between the extension body 40 and the guide tube 42 constitutes an outer diameter changing portion 43 increased in diameter in a flare shape in the distal direction.

Thus, the extension body 40 is made thinner than the base 36 and the guide tube 42. Consequently, on an outer peripheral portion of the grip 16, a constricted portion 40a of a shape recessed radially inward is formed by the extension body 40 between the base 36 (finger-rests) and the guide tube 42.

A step formed by the difference in outer diameter between the proximal end of the guide tube 42 and the distal end of the extension body 40 prevents the user's fingers holding the extension body 40 from sliding in the distal direction when the user presses the syringe 12 in the distal direction to insert the puncture needle 26 of the syringe 12 into the skin of the patient.

As shown in Figs. 1 and 3, the guide tube 42 is provided with a plurality of side holes 50 through which to expose the barrel 18 to make the inside of the barrel 18 (the liquid medicine M with which the barrel 18 is filled) visible. The side holes 50 are provided proximally relative to the annular guide 48. The side holes 50 extend through a peripheral wall constituting the guide tube 42 in the thickness direction. The side holes 50 are spaced in the circumferential direction. In the present embodiment, two side holes 50 are provided in positions opposite to each other with respect to the axis of the barrel 18.

The side holes 50 have a form of an elongated hole extending in the axial direction. The side holes 50 are formed to allow visual recognition of an area in the barrel 18 filled with the liquid medicine M across the entire axial range in the initial state of the syringe assembly 10 (a state before the discharge of the liquid medicine). Specifically, the side holes 50 are formed from a position corresponding to the distal end of the barrel body 23 to a position corresponding at least to the distal end of the gasket 22 in the initial position.

In order to provide adequate visibility of the liquid medicine M while maintaining necessary strength for the guide tube 42, the opening width W of the side holes 50 (the width along the circumferential direction of the barrel 18) in Fig 1 is set, for example, to 1 to 11 mm, and is preferably set to 5 to 7 mm when the inner diameter of the barrel 18 is 6.3 mm.

The constituent material of the grip 16 is not particularly limited, and may be, for example, a resin material such as polypropylene, polycarbonate, polyamide, polysulfone, polyarylate, ABS, or high-density polyethylene.

Next, the function of the syringe assembly 10 configured as described above will be described.

When administering the liquid medicine M to a patient, the user (or the patient himself) first connects the plunger 30 to the gasket 22 to bring it into the state shown in Fig. 1. Note that the syringe assembly 10 with the plunger 30 connected to the gasket 22 in advance as shown in Fig. 1 may be provided to the user.

Then, the user holds and pulls the distal end portion of the cap 34, thereby pulling the cap 34 out of the cover member 32 and removing the cap 34 from the puncture needle 26. At this point in time, the cover member 32 is biased to the initial position (pre-puncture position) by the elastic biasing force of the above-described spring member 33 (see Fig. 2), keeping the puncture needle 26 surrounded by the cover member 32 up to the needle point 27 at the distal end. Thus, the puncture needle 26 is prevented from being inadvertently stuck into the user or the like.

Next, the user places two fingers (for example, the index finger and the middle finger) on the pair of finger-rests 38, and holds the grip 16, putting the extension body 40 (the constricted portion 40a) between the two fingers. Then, the user brings the distal end of the cover member 32 into contact with a puncture site (arm or the like) of the patient for positioning, and further presses the barrel 18 via the grip 16. Then, the cover member 32 is displaced in the proximal direction relative to the barrel 18 against the elastic biasing force of the spring member 33.

With this relative displacement, as shown in Fig. 5, the puncture needle 26 held by the needle holder 24 is exposed from the distal opening 32a of the cover member 32, and the cover member 32 moves to the most proximal position (retracted position) in the movable range relative to the barrel 18. At this time, the cover member 32 moves in the proximal direction in the annular space S2 (see Fig. 2) formed between the barrel body 23 and the guide tube 42. This retraction movement of the cover member 32 causes most of the puncture needle 26 to be exposed from the distal end of the cover member 32 and inserted into the patient's body.

With the puncture needle 26 inserted in the patient, the user presses the plunger 30 in the distal direction. This causes the liquid medicine M with which the barrel 18 is filled to be discharged from the puncture needle 26 through the lumen of the puncture needle 26. As a result, the liquid medicine M is administered to the patient.

After the administration of the liquid medicine M, the cover member 32 is pulled away from the patient together with the prefilled syringe 12A. At this time, the spring member 33 disposed in the cover member 32 extends by the elastic restoring force, and biases the cover member 32 in the distal direction relative to the barrel 18. Consequently, as shown in Fig. 6, the cover member 32 is displaced in the distal direction relative to the barrel 18, moving from the retracted position to the locked position. In a stage where the cover member 32 has reached the locked position, the puncture needle 26 is covered by the cover member 32 up to the needle point 27, and the displacement of the cover member 32 in the proximal direction relative to the barrel 18 is prevented. Therefore, even after the administration of the liquid medicine M to the patient, inadvertent puncture can be avoided.

In this case, the syringe assembly 10 according to the present embodiment has the following effects.

According to the syringe assembly 10, the guide tube 42 of the grip 16 supports the cover member 32 of the protective device 14 from outside, and thus can prevent the cover member 32 from shaking (being unstable with the axis of the cover member 32 inclined with respect to the axis of the barrel 18) when the cover member 32 moves in the proximal direction relative to the barrel 18. That is, the cover member 32 is not only supported (guided) by the outer peripheral surface of the barrel body 23, but also supported (guided) by the inner peripheral surface of the guide tube 42 of the grip 16. Thus, the cover member 32 is supported from both inside and outside, and thus is effectively prevented from shaking with respect to the barrel body 23.

According to the syringe assembly 10, the cover member 32 can be prevented from shaking with respect to the barrel body 23 without being extended in length in the proximal direction. This can provide an adequate area that can be held by the user at the time of use. That is, the user can use the guide tube 42 as a holding area. This allows for various ways of holding, and does not deteriorate ease of use even when a structure for preventing the shaking of the cover member 32 is provided. Thus, according to the syringe assembly 10 of the present invention, the cover member 32 can be prevented from shaking without degrading ease of use.

The guide tube 42 covers the cover member 32 around its entire circumference at least when supporting and guiding the cover member 32 from outside. This configuration can prevent shaking of the cover member 32 from all directions around it.

The guide tube 42 is provided with the side holes 50 through which to expose the barrel 18 to make the inside of the barrel 18 visible. In the syringe assembly 10, it is not necessary to lengthen the cover member 32 in the proximal direction to prevent its shaking, so that degradation in the visibility of the liquid medicine caused by the cover member 32 does not occur. Further, the provision of the side holes 50 in the guide tube 42 can facilitate the visual recognition of the liquid medicine M in the barrel 18. Thus, the cover member 32 can be prevented from shaking without degrading the visibility of the liquid medicine.

The side holes 50 are spaced in the circumferential direction. This can improve the visibility of the liquid medicine.

As shown in Fig. 2, the most distal end of the guide tube 42 is located distally relative to the most proximal end of the cover member 32 in the initial state of the protective device 14. This configuration can favorably prevent the cover member 32 from shaking from the beginning of pressing the cover member 32 against an object to be punctured since the guide tube 42 covers at least part of the cover member 32 in the initial state of the protective device 14.

As shown in Fig. 5, the most distal end of the guide tube 42 is located in the axial position substantially the same as the position of the distal end face of the cover member 32 when the cover member 32 is displaced most in the proximal direction relative to the barrel 18 by being pressed against an object to be punctured. This configuration allows the length of the guide tube 42 to be increased to the maximum extent possible without impairing the function of the protective device 14. Thus, the cover member 32 can be more effectively prevented from shaking.

The proximal end of the guide tube 42 is located proximally relative to the proximal end position of the cover member 32 when the cover member 32 is displaced most in the proximal direction relative to the barrel 18 by being pressed against an object to be punctured. This configuration can reliably prevent the shaking of the cover member 32 until the cover member 32 is displaced most in the proximal direction, and can reliably provide an area that can be held by the user at the time of use.

The outer diameter of the proximal end of the guide tube 42 is larger than the outer diameter of the distal end of the extension body 40. This configuration forms a step due to the difference in outer diameter between the proximal end of the guide tube 42 and the distal end of the extension body 40. This can effectively prevent the user's fingers holding the extension body 40 from sliding in the distal direction when pressing the syringe assembly 10 in the distal direction to insert the puncture needle 26 into an object to be punctured.

As shown in Fig. 4, the grip 16 has the engaging projections 44 that engage the proximal end face of the flange 28 provided at the proximal end of the barrel 18, thereby restricting the displacement of the grip 16 in the distal direction relative to the barrel 18. This configuration can effectively prevent the grip 16 from being displaced in the distal direction relative to the barrel 18.

The engaging projections 44 are provided at the protruding pieces 46 that protrude in the proximal direction from the proximal end face of the base 36. This configuration allows the engaging projections 44 to easily elastically deform outward when the grip 16 is fitted onto the barrel 18. Thus, the grip 16 can be easily fitted onto the barrel 18.

Note that the syringe 12 is not limited to the prefilled syringe 12A, and may be a syringe filled with the liquid medicine M after product offering.

Further, the extension body 40 may not be cylindrical, and may be formed in a pillar shape extending in parallel from the base 36 and connected to the proximal end of the guide tube 42, for example. In this case, the extension body 40 is preferably formed as a pair of pillar-shaped portions.

The present invention is not limited to the above-described embodiment, and various modifications can be made without departing from the scope of the present invention.

## Claims

1. A syringe assembly (10) comprising:
a syringe (12) having a puncture needle (26) with a needle point (27) and a barrel (18) holding the puncture needle (26) ;
a hollow cylindrical grip (16) fitted on an outer peripheral portion of the barrel (18); and
a protective device (14) having a hollow cylindrical cover member (32) axially displaceable relative to the barrel (18), the protective device (14) being configured such that the cover member (32) covers at least part of the puncture needle (26) in an initial state, the cover member (32) is displaced in a proximal direction relative to the barrel (18) by being pressed against an object to be punctured when the puncture needle (26) is inserted into the object to be punctured, and the cover member (32) covers the needle point (27) after insertion of the puncture needle (26) into the object to be punctured,
wherein the grip (16) has a base (36) provided with outwardly protruding finger-rests (38), an extension body (40) protruding from the base (36) in a distal direction, and a guide tube (42) extending from the extension body (40) in the distal direction and supporting and guiding the cover member (32) from outside when the cover member (32) is axially displaced relative to the barrel (18), and
the guide tube (42) has an inner peripheral surface that can abut an outer peripheral surface of the cover member (32) in the initial state.

2. The syringe assembly (10) according to claim 1, wherein
the guide tube (42) covers the cover member (32) around an entire circumference thereof at least when supporting and guiding the cover member (32) from outside.

3. The syringe assembly (10) according to claim 1 or 2, wherein
the guide tube (42) is provided with at least one side hole (50) through which to expose the barrel (18) to make an inside of the barrel (18) visible.

4. The syringe assembly (10) according to claim 3, wherein
the at least one side hole (50) comprises a plurality of side holes (50) spaced in a circumferential direction.

5. The syringe assembly (10) according to any one of claims 1 to 4, wherein
a most distal end of the guide tube (42) is located distally relative to a most proximal end of the cover member (32) in the initial state of the protective device (14).

6. The syringe assembly (10) according to any one of claims 1 to 5, wherein
a most distal end of the guide tube (42) is located in an axial position substantially the same as a position of a distal end face of the cover member (32) when the cover member (32) is displaced most in the proximal direction relative to the barrel (18) by being pressed against the object to be punctured.

7. The syringe assembly (10) according to any one of claims 1 to 6, wherein
a proximal end of the guide tube (42) is located proximally relative to a proximal end position of the cover member (32) when the cover member (32) is displaced most in the proximal direction relative to the barrel (18) by being pressed against the object to be punctured.

8. The syringe assembly (10) according to any one of claims 1 to 7, wherein
an outer diameter of a proximal end of the guide tube (42) is larger than an outer diameter of a distal end of the extension body (40).

9. The syringe assembly (10) according to any one of claims 1 to 8, wherein
the grip (16) has engaging projections (44) that engage a proximal end face of a flange (28) provided at a proximal end of the barrel (18), thereby restricting displacement of the grip (16) in the distal direction relative to the barrel (18).

10. The syringe assembly (10) according to claim 9, wherein
the engaging projections (44) are provided at protruding pieces (46) protruding from a proximal end face of the base (36) in the proximal direction.

11. The syringe assembly (10) according to any one of claims 1 to 10, further comprising:
a gasket (22) slidably inserted in the barrel (18);
a plunger (30) connected to the gasket (22) or connectable to the gasket (22); and
a liquid medicine (M) with which a liquid chamber formed by the barrel (18) and the gasket (22) is filled.
